(19) Europäisches Patentamt
European Patent Office
Office européen des brevets

(11) EP 2 324 836 A1

(12) **EUROPEAN PATENT APPLICATION**
published in accordance with Art. 153(4) EPC

(43) Date of publication:
25.05.2011 Bulletin 2011/21

(51) Int Cl.:
*A61K 31/695* (2006.01)    *A61K 31/4412* (2006.01)
*A61P 35/00* (2006.01)    *A61P 43/00* (2006.01)

(21) Application number: 09814281.3

(22) Date of filing: 15.09.2009

(86) International application number:
PCT/JP2009/004599

(87) International publication number:
WO 2010/032436 (25.03.2010 Gazette 2010/12)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA RS

(30) Priority: 16.09.2008 JP 2008236643

(71) Applicant: Taiho Pharmaceutical Co., Ltd.
Chiyoda-ku, Tokyo 1018444 (JP)

(72) Inventor: MURAKAMI, Koji
Tokyo 101-8444 (JP)

(74) Representative: Hartz, Nikolai
Wächtershäuser & Hartz
Patentanwaltspartnerschaft
Weinstrasse 8
80333 München (DE)

(54) **ANTITUMOR AGENT CONTAINING 4-[[3,5-BIS(TRIMETHYLSILYL)BENZOYL]AMINO¨BENZOIC ACID**

(57)    Disclosed is a novel combination therapy of sorafenib having a remarkable antitumor effect. Specifically disclosed is an antitumor agent obtained by combining sorafenib or a salt thereof with 4-[[3,5-bis(trimethylsilyl)benzoyl]amino]benzoic acid or a salt thereof.

FIG. 1

**:p<0.01, ***:p<0.001

## Description

Technical Field

The present invention relates to a novel antitumor agent containing sorafenib or a salt thereof and 4-[[3,5-bis (trimethylsilyl)benzoyl]amino]benzoic acid or a salt thereof.

Background Art

A raf kinase protein, which is a product of oncogene raf, transmits signals of growth factor receptors to the MAP kinase cascade, and through the activation of the kinase cascade, accelerates the proliferation of cancer cells. Insulin-like Growth Factor-II (IGF-II) is a characteristic proliferation factor which is increasingly expressed in many cancer cells including liver cancer, it is known that an increase in the signals of type I IGF receptors, which are the receptors of the foregoing Insulin-like Growth Factor, causes activation of the raf kinase cascade. It is known that a reduction in endogenous raf kinase inhibitor proteins accelerates the onset of cancer. Furthermore, it is reported that increased expression of Vascular Endothelial Growth factor (VEGF), which is a representative angiogenesis factor and is one of the targets for cancer treatment, is correlated with the poor prognosis of liver cancer patients. Therefore, a multikinase inhibitor drug which has both an inhibitory action against raf kinases and an inhibitory action against VEGFR kinases, N-(4-chloro-3-(Trifluoromethyl)phenyl)-N'-(4-(2-(N-methylcarbamoyl)-4-pyridyloxy)phenyl)urea (hereinafter, sorafenib), has been approved as a therapeutic drug for renal cancer and liver cancer (Patent Document 1 and Non-Patent Document 2).

Furthermore, in order to obtain new antitumor effects, attempts have also been made to use sorafenib or a salt thereof and another antitumor agent such as gemcitabine or doxorubicin in combination (Patent Documents 2 and 3, and Non-Patent Documents 2 and 3).

However, satisfactory antitumor effects have not been hitherto obtained, and there is a strong demand for a novel combination therapy of sorafenib or a salt thereof exhibiting more potent antitumor effects.

Prior Art Documents

Patent Documents

Patent Document 1: JP-B-3845792
Patent Document 2: JP-A-2005-511658
Patent Document 3: WO 2006/125539

Non-Patent Documents

Non-Patent Document 1: Nature Reviews Drug Discovery, 2006; 5(10):835-44.
Non-Patent Document 2: Annals of Oncology, 2006; 17(5):866-873.
Non-Patent Document 3: Clinical Cancer Research, 2006; 12 (1) :144-51.

Summary of the Invention

Problem to be Solved by the Invention

The present invention is to provide a novel combination therapy of sorafenib or a salt thereof exhibiting remarkable antitumor effects.

Means for Solving the Problem

Under such circumstances, the inventors of the present invention conducted extensive research on a novel combination therapy of sorafenib or a salt thereof and another antitumor agent, in order to develop a cancer treatment method that further contributes to the prolongation of the survival time of patients. As a result, the inventors found that when 4-[[3,5-bis(trimethylsilyl)benzoyl]amino]benzoic acid (hereinafter, referred to as "compound 1"), which is a RARα-selective agonist compound, is used in combination with sorafenib or a salt thereof, a synergistic antitumor effect is obtained, and this antitumor effect is remarkably excellent as compared with the antitumor effects obtainable by known combination therapies using gemcitabine, doxorubicin or the like.

The compound 1 is a drug showing a mechanism of action that is different from that of those drugs whose synergistic antitumor effects resulting from a combined use with sorafenib have been previously confirmed. It is quite surprising that a combined use of sorafenib and the compound 1 provides a stronger antitumor effect as compared with the conventional combined use of antitumor agents.

Specifically, The present invention is related to following items 1) to 6):

1) An antitumor agent comprising sorafenib or a salt thereof and 4-[[3,5-bis (trimethylsilyl) benzoyl] amino]benzoic acid or a salt thereof in combination.

2) The antitumor agent according to item 1), which is a combination preparation.

3) The antitumor agent according to item 1) or 2), which is a kit comprising a preparation containing sorafenib or a salt thereof and a preparation containing 4-[[3,5-bis(trimethylsilyl)benzoyl]amino]benzoic acid or a salt thereof.

4) Use of a combination of sorafenib or a salt thereof and 4- [[3,5-bis(trimethylsilyl)benzoyl] amino] benzoic acid or a salt thereof, for the preparation of an antitumor agent.

5) A method for treating cancer, the method comprising administering to a patient a combination of sorafenib or a salt thereof and 4- [[3, 5-bis (trimethylsilyl) benzoyl] amino] benzoic acid or a salt thereof.

6) The method for treating cancer according to item 5), wherein sorafenib or a salt thereof and 4-[[3,5-bis(trimethylsilyl) benzoyl]amino]benzoic acid or a salt thereof are administered simultaneously or separately at an interval.

Effect of the Invention

The antitumor agent of the present invention containing sorafenib or a salt thereof and the compound 1 or a salt thereof provides remarkably high antitumor effects, for example, a decrease in the tumor volume and stagnation of tumor growth, as compared with the conventional combined use of antitumor agents. Therefore, a longer survival time can be obtained by using the antitumor agent of the present invention.

Brief Description of the Drawings

FIG. 1 is a graph showing the therapeutic effects of a combination therapy of sorafenib and compound 1;

FIG. 2 is a graph showing the therapeutic effects of a combination therapy of sorafenib and doxorubicin; and

FIG. 3 is a graph showing the therapeutic effects of a combination therapy of sorafenib and gemcitabine.

Detailed Description of the Invention

Sorafenib of the present invention is a known compound represented by N-(4-chloro-3-(trifluoromethyl)phenyl)-N'-(4-(2-(N-methylca rbamoyl)-4-pyridyloxy)phenyl)urea, and is known to provide antitumor effects against cancers such as liver cancer and renal cancer according to inhibition of Raf kinases or VEGFR kinases.

The salt of sorafenib is not limited as long as the salt is a pharmaceutically acceptable salt, and examples thereof include salts of inorganic acids and organic acids such as hydrochloric acid, hydrobromic acid, sulfuric acid, phosphoric acid, methanesulfonic acid, trifluoromethanesulfonic acid, benzenesulfonic acid, p-toluenesulfonic acid, 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid and mandelic acid, while a salt of p-toluenesulfonic acid is preferred.

Sorafenib or a salt thereof can be produced by a known method, for example, a method described in Japanese Patent No. 3845792, and commercially available medicines such as Nexavar (registered trademark, manufactured by Bayer Pharmaceuticals Corp.) may also be used.

Compound 1 of the present invention is a known RARα-selective agonist compound represented by 4- [[3,5-bis (trimethylsilyl) benzoyl] amino] benzoic acid, and is known to provide antitumor effects against numerous types of cancer such as liver cancer and lung cancer (J. Clin. Oncol. 2002, 20(16), 3522-3532; Japanese Patent No. 2761023).

The salt of the compound 1 is not particulary limited as long as the salt is a pharmaceutically acceptable salt, and examples thereof include salts of metals such as sodium, potassium and calcium; ammonium salts; and salts of organic acids such as methylamine, ethylamine, trimethylamine, triethylamine, pyridine, picoline, arginine and lysine. The compound 1 or a salt thereof can be produced by a known method, for example, a method described in Japanese Patent No. 2761023.

As will be shown in the Examples that are described below, when sorafenib or a salt thereof and the compound 1 or a salt thereof are administered in combination, the agents exhibit a remarkably strong antitumor effect compared with the case where the agents are administered individually. Therefore, a preparation containing sorafenib or a salt thereof and the compound 1 or a salt thereof as active ingredients, is useful as an antitumor agent.

There are no particular limitations on the cancer that can be treated by the antitumor agent of the present invention, and examples of the cancer include liver cancer, renal cancer, head and neck cancer, esophagus cancer, stomach cancer, colon cancer, rectum cancer, gall bladder/biliary duct cancer, pancreas cancer, lung cancer, breast cancer, ovary cancer, cervical cancer, uterine cancer, urinary bladder cancer, prostate cancer, testicular cancer, osteo/chondrosarcoma, leukemia, malignant lymphoma, multiple myeloma, skin cancer, brain tumor, and mesothelioma. Preferred examples include liver cancer, renal cancer and lung cancer.

The antitumor agent of the present invention may be prepared as a combination preparation, by formulating the sorafenib or a salt thereof and the compound 1 or a salt thereof in their respective effective amounts, into a single preparation at an appropriate mixing ratio (single preparation form), or may be prepared by formulating individual preparations respectively containing the respective ingredients in their respective effective amounts, so that the preparations can be used simultaneously or separately at an interval (two-preparation form).

The dosage form of the preparation is not particulary limited, and the dosage form can be optionally selected according to the purpose of treatment. Specific examples include oral preparations (tablets, coated tablets, powders, granules, capsules, liquids, and the like), injectable preparations, suppositories, patches, and ointments. The dosage form of Sorafenib or a salt thereof and the compound 1 or a salt thereof may be different or the same.

A preparation containing sorafenib or a salt thereof and/or the compound 1 or a salt thereof according to the present invention, can be prepared by a conventionally known method using a pharmaceutically acceptable carrier. Examples of such a carrier include various carriers that are generally used in conventional drugs, such as an excipient, a binder, a disintegrant, a gliding agent, a diluent, a dissolution aid, a suspending agent, an isotonic agent, a pH adjusting agent, a buffering agent, a stabilizer, a colorant, a flavoring agent, and an odor improving agent.

Examples of the excipient include lactose, sucrose, sodium chloride, glucose, maltose, mannitol, erythritol, xylytol, maltitol, inositol, dextran, sorbitol, albumin, urea, starch, calcium carbonate, kaolin, crystalline cellulose, silicic acid, methylcellulose, glycerin, sodium alginate, gum arabic and mixtures thereof. Examples of the gliding agent include purified talc, stearic acid salts, borax, polyethylene glycol and mixtures thereof. Examples of the binder include simple syrup, glucose liquid, starch liquid, gelatin solution, polyvinyl alcohol, polyvinyl ether, polyvinylpyrrolidone, carboxymethyl cellulose, shellac, methyl cellulose, ethyl cellulose, water, ethanol, potassium phosphate and mixtures thereof. Examples of the disintegrant include dried starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, lactose and mixtures thereof. Examples of the diluent include water, ethyl alcohol, macrogol, propylene glycol, ethoxylated isostearyl alcohol, polyoxylated isostearyl alcohol, polyoxyethylene sorbitan fatty acid esters and mixtures thereof. Examples of the stabilizer include sodium pyrosulfite, ethylenediamine tetraacetic acid, thioglycolic acid, thiolactic acid and mixtures thereof. Examples of the isotonic agent include sodium chloride, boric acid, glucose, glycerin and mixtures thereof. Examples of the pH adjusting agent and buffering agent include sodium citrate, citric acid, sodium acetate, sodium phosphate and mixtures thereof. Examples of a soothing agent include procaine hydrochloride, lidocaine hydrochloride and mixtures thereof.

Furthermore, the amount of sorafenib or a salt thereof and the compound 1 or a salt thereof in the preparation is preferably such that the amount of sorafenib or a salt thereof in the preparation is generally 1 to 1000 mg in terms of sorafenib, and the amount of the compound 1 or a salt thereof is 0.001 to 1000 mg.

When the antitumor agent of the present invention is provided as a kit, the kit can be designed such that the preparations containing sorafenib or a salt thereof and the compound 1 or a salt thereof formulated as described above, are separately packaged, and the medicine preparations are taken out from the respective packages and used at the time of administration. Furthermore, the respective medicine preparations may also be packaged in a form appropriate for combined administration for each dose.

The dosage amount of sorafenib or a salt thereof and the compound 1 or a salt thereof according to the present invention is not particulary limited, as long as it is an amount that allows sorafenib or a salt thereof and the compound 1 or a salt thereof to exhibit an antitumor effect in a synergistic manner and to effectively treat cancer. The amount of administration is appropriately adjusted in accordance with the age of the patient, type of cancer, stage of disease, presence or absence of metastasis, backgroundof treatment, presence or absence of other antitumor agents, and the like. The dosage amount of sorafenib or a salt thereof is, for example, about 1 to 5000 mg/day in terms of sorafenib, and the dosage amount of the compound 1 or a salt thereof is about 0.05 to 5000 mg/day. A preferred dosage amount of sorafenib or a salt thereof is about 50 to 2000 mg/day in terms of sorafenib, and a preferred dosage amount of the compound 1 or a salt thereof is about 0.1 to 1000 mg/day.

The order of administration or interval length of administration of sorafenib or salt thereof and the compound 1 or a salt thereof according to the present invention is not particulary limited, as long as the order of administration or the interval of administration is in the range in which a synergistic effect can be obtained. Furthermore, in the case of using the agents as a kit, the respective individual preparations may be administered simultaneously or separately at an interval.

Examples

Hereinafter, the present invention will be described in more detail by way of Pharmacological Test Examples and Comparative Examples. However, these Examples should not be construed to limit the scope of the subject invention.

As the solvent for the administration of various drugs in the following Test Examples, 0.5% hydroxypropylmethyl cellulose (Shin-Etsu Chemical Co., Ltd.) was used for the compound 1, and a mixed liquid of 2.5% of dimethyl acetamide (Wako Pure Chemical Industries, Ltd.), 7.5%ofCremophor (Nacalai Tesque, Inc.) and 90% of 0.1 N hydrochloric acid (Wako Pure Chemical Industries, Ltd.) was used for sorafenib. A set of one-week doses of the compound 1 was prepared and was stored in refrigeration in the dark. Sorafenib was weighed at every time of use, and a liquid for administration was prepared. Furthermore, human liver cancer cells JHH-7 which exhibited high expression of IGF-II and RARα were subcultured and maintained in a $CO_2$ incubator using ASF medium 104 (Ajinomoto Co., Inc.). BALB/cA Jcl-nu male nude mice (Clea Japan, Inc.) were used as the efficacy test animals.

[Pharmacological Test Example 1] Combination treatment effect of sorafenib and compound 1

Human liver cancer cell line JHH-7 composed of cells exhibiting high expression of RARα, was transplanted in an amount of $1 \times 10^6$ cells (0.02 mL) for each animal, into the left lateral liver lobe of a male nude mouse which was under nembutal anesthesia. On the 11th day after the transplantation, α-fetoprotein (AFP) in the blood was quantified (Diagnostic Automation, Inc., Cat. No. : 5106-16) as an index of tumor implantation, and the animals were grouped by stratified random allocation into seven animals per group so as to make the average AFP value uniform. The compound 1 and sorafenib were orally administered once a day for 14 consecutive days. The compound 1 was administered in an amount of 2 mg/kg, which is the maximum non-toxic dose allowing verification of the effectiveness in mouse. Sorafenib was administered in an amount of 10 mg/kg, which reaches a blood concentration close to that obtainable by administration of 400 mg, which is the clinical maximum dose. On the 15th day after the initiation of administration, the mice were killed by bleeding under ether anesthesia, subsequently tumors formed in the left lateral liver lobe were extracted, and the weight of the tumors was measured. The tumor growth rate (%) was calculated by the formula shown below, and the T/C value of each of the drug administered groups was calculated, while the average tumor weight of the non-drug administered control group was taken as 100%. This T/C value was used as an index of the antitumor effect. The statistical significance of the single drug administered groups was determined by Welch's two-tailed test, and the statistical significance of the effect synergizing action obtained by combined use was determined by the intersection union test based on Welch's two-tailed test.
$$\texttt{Tumor growth rate (\%) = T/C} \times \texttt{100}$$

T: Average tumor weight of a group with administration of the test compound
C: Average tumor weight of a group without administration of the test compound

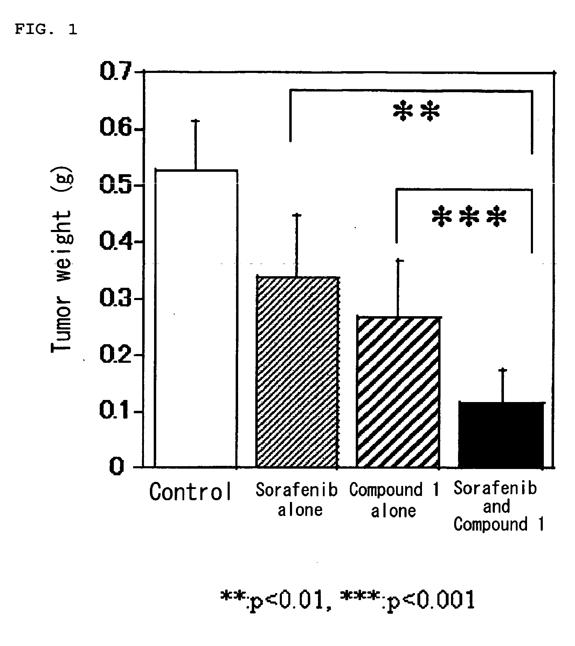

The results are shown in FIG. 1. The T/C values of the sorafenib single drug administered group and the compound 1 single drug administered group were 65% and 51%, respectively, and thus a significant therapeutic effect, as compared with the control group, was confirmed. On the other hand, the T/C value of the sorafenib and compound 1 combination group was 22%. The therapeutic effect of the combination group was statistically significant even when compared with the respective single drug administered groups, and an obvious synergism of activity was confirmed. In addition, it was also confirmed that this combination effect is similar to that of using tosylate salt that is in clinical use.

[Comparative Example 1] Combination treatment effect of sorafenib and doxorubicin

In the same manner as in the Pharmacological Test Example 1, human liver cancer cell line JHH-7 was transplanted in an amount of $1 \times 10^6$ cells (0.02 mL) for each animal, into the left lateral liver lobe of a male nude mouse which was under nembutal anesthesia. On the 11th day after the transplantation, the animals were grouped into seven animals per group, and the treatment experiment was initiated. Doxorubicin was administered such that 10 mg/kg, which is the maximum tolerated dose for a mouse, was administered one time intravenously. Sorafenib was orally administered once a day for 14 consecutive days. On the 15 th day after the initiation of administration, the mice were killed by bleeding under ether anesthesia, subsequently tumors formed in the left lateral liver lobe were extracted, weighed and evaluated as in the Pharmacological Test Example 1 was made.

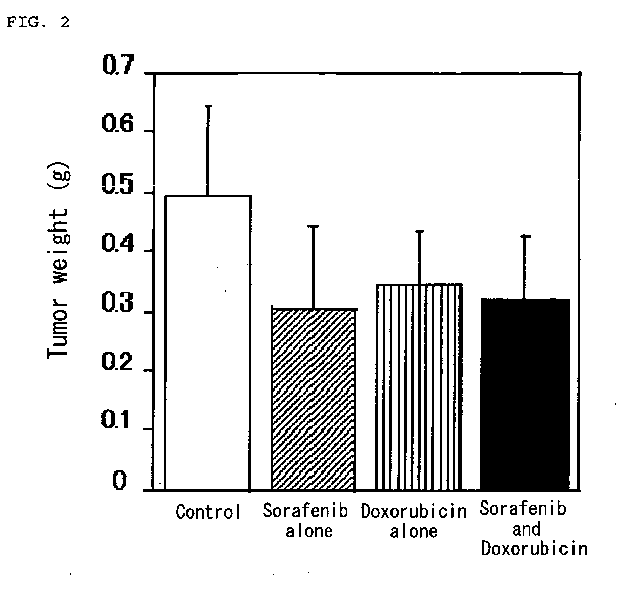

The results are presented in FIG. 2. In the present test, the T/C values of the sorafenib single drug administered group and the doxorubicin single drug administered group were 62% and 70%, respectively. On the other hand, even though sorafenib and doxorubicin were used in combination, the T/C value was 65%, and the therapeutic effect was not substantially different from that of the sorafenib single drug administered group. Furthermore, the statistical significance was not found, and an obvious synergism of activity was not recognized.

[Comparative Example 2] Combination treatment effect of sorafenib and gemcitabine

In the same manner as in the Pharmacological Test Example 1, human liver cancer cell line JHH-7 was transplanted in an amount of $1 \times 10^6$ cells (0.02 mL) for each animal, into the left lateral liver lobe of a male nude mouse which was under nembutal anesthesia. On the 11th day after the transplantation, the animals were grouped into seven animals per group, and the treatment experiment was initiated. Gemcitabine was administered such that 120 mg/kg, which is the maximum tolerated dose for a mouse, was administered intraperitoneally four times in total at an interval of 3 days. Sorafenib was orally administered once a day for 14 consecutive days. On the 15th day after the initiation of administration, the mice were killed by bleeding under ether anesthesia, subsequently tumors formed in the left lateral liver lobe were extracted, weighed and evaluated as in the Pharmacological Test Example 1 was made.

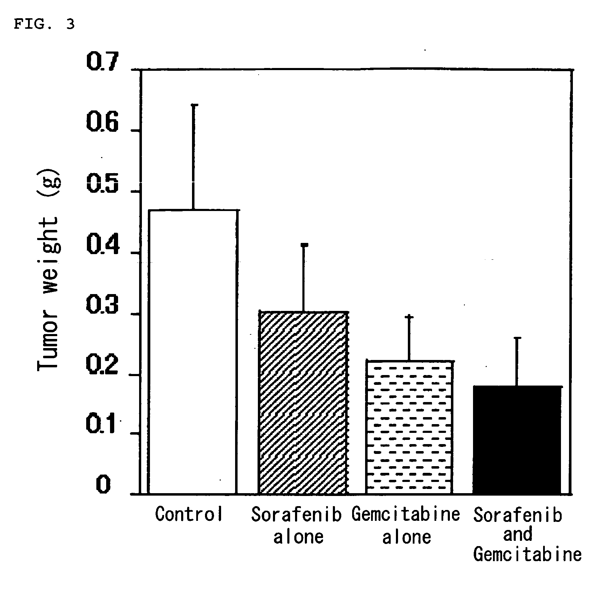

The results are shown in FIG. 3. In the present test, the T/C values of the sorafenib single drug administered group and the gemcitabine single drug administered group were 60% and 47%, respectively. The T/C value of the sorafenib and gemcitabine combination group was 38%. Similarly to the case of the combination of sorafenib and doxorubicin, an obvious effect synergizing action was not recognized.

**Claims**

1. An antitumor agent comprising sorafenib or a salt thereof and 4- [[3,5-bis (trimethylsilyl) benzoyl] amino] benzoic acid or a salt thereof in combination.

2. The antitumor agent according to claim 1, which is a combination preparation.

3. The antitumor agent according to claim 1 or 2, which is a kit comprising a preparation containing sorafenib or a salt thereof and a preparation containing 4-[[3,5-bis(trimethylsilyl)benzoyl]amino]benzoic acid or a salt thereof.

4. Use of a combination of sorafenib or a salt thereof and 4- [[3, 5-bis (trimethylsilyl) benzoyl] amino] benzoic acid or a salt thereof, for the preparation of an antitumor agent.

5. A method for treating cancer, the method comprising administering to a patient a combination of sorafenib or a salt thereof and 4- [[3,5-bis (trimethylsilyl) benzoyl] amino] benzoic acid or a salt thereof.

6. The method for treating cancer according to claim 5, wherein sorafenib or a salt thereof and 4-[[3,5-bis(trimethylsilyl)benzoyl]amino]benzoic acid or a salt thereof are administered simultaneously or separately at an interval.

FIG. 1

**:p<0.01, ***:p<0.001

FIG. 2

FIG. 3

<table>
<tr><td colspan="2" align="center">**INTERNATIONAL SEARCH REPORT**</td><td colspan="2">International application No.<br>PCT/JP2009/004599</td></tr>
</table>

A. CLASSIFICATION OF SUBJECT MATTER
A61K31/695(2006.01)i, A61K31/4412(2006.01)i, A61P35/00(2006.01)i,
A61P43/00(2006.01)i

According to International Patent Classification (IPC) or to both national classification and IPC

B. FIELDS SEARCHED

Minimum documentation searched (classification system followed by classification symbols)
A61K31/695, A61K31/4412, A61P35/00, A61P43/00

Documentation searched other than minimum documentation to the extent that such documents are included in the fields searched
| | | |
|---|---|---|
| Jitsuyo Shinan Koho | 1922-1996 | Jitsuyo Shinan Toroku Koho 1996-2009 |
| Kokai Jitsuyo Shinan Koho | 1971-2009 | Toroku Jitsuyo Shinan Koho 1994-2009 |

Electronic data base consulted during the international search (name of data base and, where practicable, search terms used)
CAP/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | Fumihiko NAKAI et al., "Kansaibogan ni Taisuru Bunshi Hyotekiyaku no Kaihatsu Jokyo", Kan-tan-sui, 2007, vol.55, no.5, pages 861 to 866 | 1-4 |
| Y | JP 2005-511658 A (Bayer Pharmaceuticals Corp.), 28 April 2005 (28.04.2005), claims 1, 6, 7, 15, 16, 20 to 22, 32, 33; paragraph [0071]; examples<br>& WO 2003/047579 A1 & US 2003/232765 A1<br>& EP 1450799 A1 & US 2006/247186 A1<br>& EP 1450799 B1 & EP 1769795 A2<br>& EP 1450799 B9 & EP 1769795 A3 | 1-4 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>06 October, 2009 (06.10.09) | Date of mailing of the international search report<br>20 October, 2009 (20.10.09) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/004599 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | Shinji OIE et al., "Hito Kansaibogan Yuraikabu JHH-7 no Bunshi Seibutsugakuteki Tokucho to RAR-α Tokuiteki Ketsugosei o Yusuru Kagobutsu TAC-101 ni Taisuru Hannosei", The Cell, 2001, vol.33, no.11, pages 428 to 431 | 1-4 |
| Y | MURAKAMI, K. et al, 4-[3,5-Bis(trimethylsilyl) benzamido] benzoic acid (TAC-101) inhibits the intrahepatic spread of hepatocellular carcinoma and prolongs the life-span of tumor-bearing animals, Clin Exp Metastasis, 1998, Vol.16, No.7, p.633-43 | 1-4 |

Form PCT/ISA/210 (continuation of second sheet) (April 2007)

<table>
<tr><td>**INTERNATIONAL SEARCH REPORT**</td><td>International application No.<br>PCT/JP2009/004599</td></tr>
</table>

**Box No. II        Observations where certain claims were found unsearchable (Continuation of item 2 of first sheet)**

This international search report has not been established in respect of certain claims under Article 17(2)(a) for the following reasons:

1. ☒ Claims Nos.: 5, 6
   because they relate to subject matter not required to be searched by this Authority, namely:
   The inventions as set forth in claims 5 and 6 include the method for treatment of the human body or animal body by surgery or therapy and thus relate to a subject matter which this International Searching Authority is (Continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable,
the                                      payment of a protest fee.

                                    ☐ The additional search fees were accompanied by the applicant's protest but the applicable protest
                                       fee was not paid within the time limit specified in the invitation.

                                    ☐ No protest accompanied the payment of additional search fees.

Form PCT/ISA/210 (continuation of first sheet (2)) (April 2007)

**INTERNATIONAL SEARCH REPORT**

International application No.

PCT/JP2009/004599

Continuation of Box No.II-1 of continuation of first sheet(2)

not required, under the provisions of Article 17(2)(a)(i) of the PCT and Rule 39.1(iv) of the Regulations under the PCT, to search.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3845792 B
- JP 2005511658 A
- WO 2006125539 A
- JP 2761023 B

**Non-patent literature cited in the description**

- *Nature Reviews Drug Discovery,* 2006, vol. 5 (10), 835-44
- *Annals of Oncology,* 2006, vol. 17 (5), 866-873
- *Clinical Cancer Research,* 2006, vol. 12 (1), 144-51
- *J. Clin. Oncol.,* 2002, vol. 20 (16), 3522-3532